# EUROPEAN PATENT APPLICATION

(11) **EP 3 677 223 A1**
(43) Date of publication of application: **08.07.2020**
(21) Application number: 19150389.5
(22) Date of filing: 04.01.2019
(51) Int. Cl.: A61F 2/24

(54) **A DEVICE FOR DETERMINING THE PLANE OF A HEART VALVE ANNULUS**

(71) Applicant: Casserley, Ivan, 7 Dublin (IE); McCluskey, Sinead, 7 Dublin (IE)
(72) Inventor: Casserley, Ivan, Dublin, 7 (IE)
(74) Representative: Purdylucey Intellectual Property

(57) **Abstract**

A device (5) for determining a plane of an aortic valve annulus (E), comprises an elongated control shaft (8) and a locating head at a distal end of the shaft. The locating head comprises three adjacent and separate cusp-locating loops (9) that are radially adjustable from a delivery configuration to a deployed trifurcated configuration where the cusp-locating loops (9, 31, 32) are angled outwardly relative to the central shaft and positioned to nest and self-centre in the cusps (D1, D2, D3) of a stenotic aortic valve during use. An apex (10) of each cusp-locating head comprises a radio-opaque material (11).

## Description

### Field of the Invention

The present invention relates to a device for determining the plane of a heart valve annulus. In particular, the invention relates to a method of determining the plane of the aortic valve annulus. Also contemplated are methods of heart valve replacement that include a step of determining the plane of a heart valve annulus during the valve replacement procedure.

### Background to the Invention

Aortic valve stenosis is a condition where the three leaflets of the aortic valve become calcified over time, resulting in restricted valve movement, restricted blood flow from the left ventricle to the aorta. Symptoms of the condition may include breathlessness, chest pain, and fainting (syncope). In addition to the symptoms, the wall of the left ventricle may also exhibit muscular thickening due to the ventricle having to work harder to pump blood through the narrow valve opening into the aorta. This results in a pressure overload in the left ventricle, which may lead to heart failure. Aortic stenosis may be diagnosed with an echocardiogram (heart ultrasound), and if diagnosed may require valve replacement.

TAVI (Transcatheter Aortic Valve Implantation) is a procedure that involves replacement of a patients' aortic valve with a synthetic replacement valve using percutaneous methods. The procedure typically involves passing a guidewire through the femoral artery, up the aorta and around the aortic arch, and through the stenotic aortic valve into the left ventricle. A replacement valve coaxially mounted on a delivery catheter is than advanced over the guidewire to the aortic valve, positioned such that the replacement valve frame is located just below the aortic annulus, and is deployed in that position.

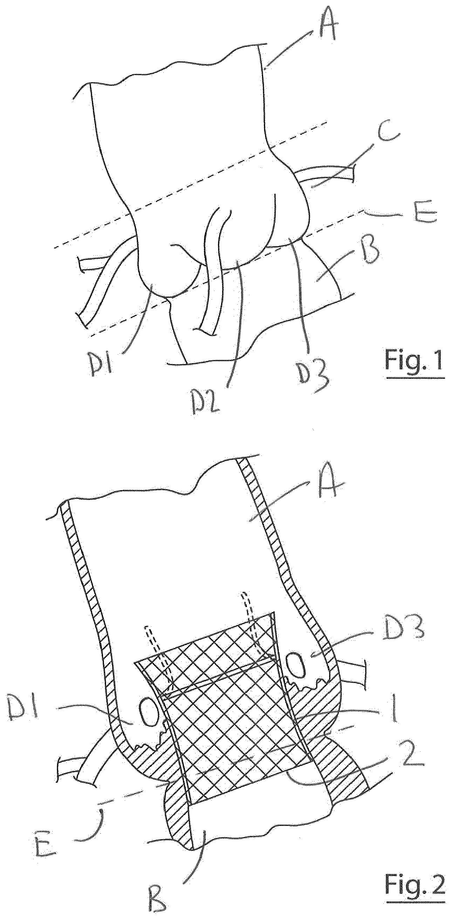

During TAVI, it is necessary to accurately define the plane of the aortic annulus to allow accurate positioning of the stent frame of the TAVI valve and optimize outcomes (**Figures 1 and 2**). This is based on the fact that the goal of implantation is to position the valve frame just below the plane of the aortic annulus. This is typically performed using a pre-procedural CT scan which requires contrast and may occasionally be inaccurate due to differences in the patient position during the pre-procedural CT scan versus the patient position on the catheterisation lab table during the TAVI procedure. The alternative to pre-procedural CT scan assessment is on-table angiographic assessment which also requires contrast and often requires multiple angiograms due to the trial and error approach required to define the optimal implantation angle using this method.

It is an object of the invention to overcome at least one of the above-referenced problems.

### Summary of the Invention

The present invention addresses the need for a device for accurately determining the plane of a heart valve annulus that can be employed during a heart valve replacement procedure and that allows the plane of the aortic valve annulus to be imaged on a catheterisation lab table under X-ray without the need for contrast. The device has an elongated control shaft and a locating head at a distal end of the arm, and is configured for transluminal delivery around the aortic arch and to the aortic valve. The device typically includes a delivery catheter, with the control shaft and locating head disposed in the catheter and configured for axial movement relative to the catheter to deploy the locating head proud of a distal end of the catheter during use. The locating head has three adjacent and separate cusp locating heads (for example loops or prongs) that are generally radially adjustable from a folded delivery configuration to a deployed trifurcated configuration where the cusp-locating heads are positioned to nest in the cusps of a stenotic aortic valve when the device is advanced towards the aortic valve. When loops are employed, the loops are preferably separate from each other (i.e. not interlaced or intertwined). The device will generally have three cusp-locating heads, although an embodiment of the device having two cusp-locating heads is envisaged for patients identified as having bicuspid valves. At least the apex of the cusp-locating head will have a radio-opaque marker that can be imaged under X-ray. In use, the device is generally inserted into a patient trans-femorally through a guide catheter, and advanced around the aortic arch until it is disposed proximal to the diseased aortic valve. The locating head is then deployed, allowing the cusp-locating heads to deploy to an outwardly angled cusp-locating position, and the device is then advanced and adjusted until the apex of each head nests in an aortic valve cusp. The use of loops as cusp-locating heads allows the device to self-centre in the cusps. Due to the radio-opaque marker at the apex of each head, the exact position of the cusps can be viewed under X-ray, without the need for any contrast agent, allowing the plane of the valve annulus to be accurately determined while the patent is on the cath-lab table. The device heads or loops are then withdrawn from the aortic valve into the guide catheter. The TAVI valve can then be delivered across the native aortic valve in the annulus plane determined by the novel device. The heads or loops of the device can then be re-advanced from the guide catheter into the aortic sinuses to confirm the location of the TAVI valve with respect to the plane of the annulus. The device heads or loops are then retrieved and the TAVI valve is deployed.

According to a first aspect of the present invention, there is provided a device for locating the plane of a heart valve annulus, comprising an elongated central shaft and a locating head at a distal end of the shaft, the locating head comprising two or three adjacent and separate cusp-locating heads that are radially adjustable from a delivery configuration to a deployed bifurcated or trifurcated configuration where the cusp-locating heads are angled outwardly relative to the central shaft and positioned to nest in the cusps of a stenotic aortic valve, wherein an apex of each cusp-locating head comprises a radio-opaque material.

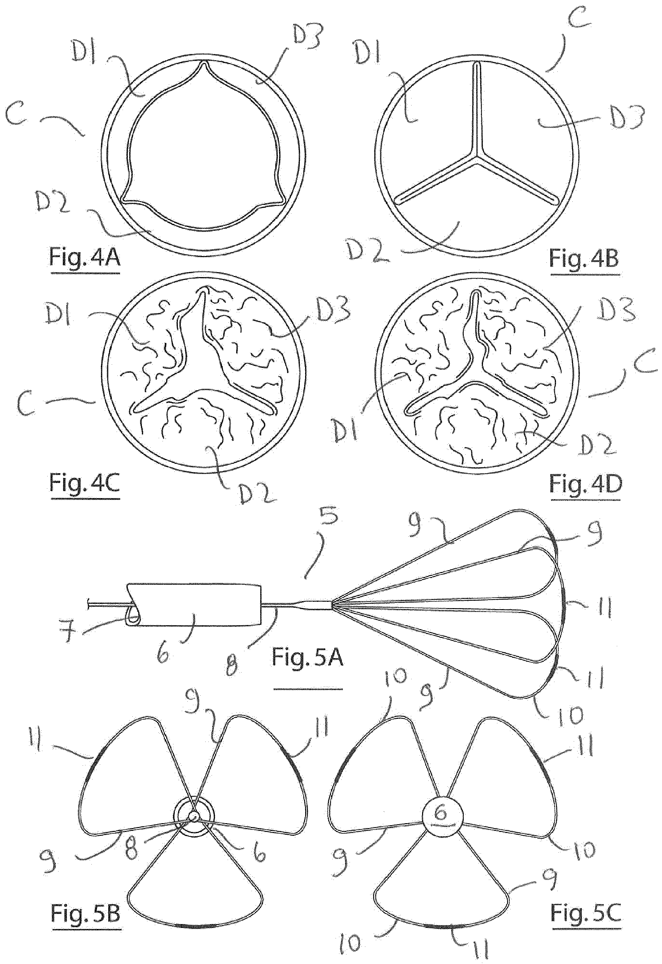

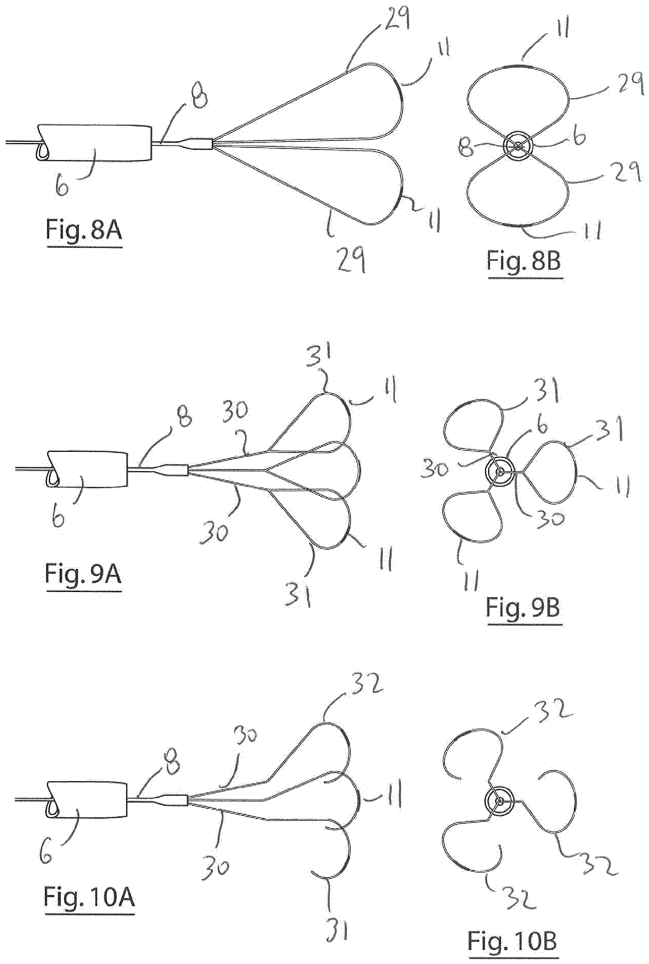

In one embodiment, the cusp-locating head comprises a loop. The loop may be a closed loop (for example Fig. 5A or 8A), or an open loop (for example, Fig. 10A). In one embodiment, the cusp-locating head comprises an extension arm and a loop or other cusp-locating element disposed at a distal end of the extension arm (for example, Figs 9 and 10). In other embodiments, the cusp-locating head may be a prong with an atraumatic head. The cusp-locating head is typically dimensioned to nestle within a heart valve cusp when the valve is open or closed.

The cusp-locating heads when deployed are generally symmetrically arranged about an axis of the elongated control shaft. The device is generally configured so that the cusp-locating heads are outwardly biased when deployed. This means that when the deployed, the cusp locating heads will bear against the wall of the aorta as the device is being advanced towards aortic valve. The cusp-locating heads are typically configured for positional adjustment with respect to each other, to allow for aortic valves having cusps that are not of uniform size or shape. The cusp-locating heads are generally designed to self-centre the locating head in the aortic sinuses - the use of loops as cusp-locating heads is ideal for this purpose.

In one embodiment, the apex of each loop when deployed is shaped to nest snugly within an aortic valve cusp. Thus, the distal end of the loop is typically dimensioned to fit within the cusp when the valve is in an open or closed position. In a preferred embodiment, the curvature of the distal end of the loop is designed to match the curvature of an aortic valve cusp, allowing the distal end of the loop nest snugly within the cusps of the aortic valve.

In one embodiment, the loops are attached at their proximal ends to the central shaft. An example of this embodiment is shown in Fig. 5.

In another embodiment, each loop is coupled to the central shaft by an extension arm. An example of this embodiment is shown in Figs 9 and 10.

In one embodiment, the device comprises an elongated catheter having a central lumen, wherein the elongated shaft and locating head are disposed within the lumen of the catheter and axially movable relative to the catheter to deploy the locating head.

In one embodiment, the locating head is formed from a shape-memory material and configured to adapt a cusp-engaging configuration when deployed. The loops may be formed from nitinol wire.

In one embodiment, the loops are angled outwardly of the central shaft at an angle of 20-50°.

In one embodiment, the radio-opaque material comprises a noble metal (for example, gold or platinum iridium). In one embodiment, the radio-opaque material is coated on to the apex of the loops.

In another aspect, the invention provides a method of determining a plane of a heart valve annulus, typically an aortic valve annulus, comprising the steps of:
advancing a device of the invention through a blood vessel to a position proximal of the heart valve;
deploying the device to expose the locating head comprising the cusp-locating heads;
advancing the control shaft and locating head until the cusp-locating heads nestle in cusps of the valve; and
taking an image of the device with the cusp-locating heads nestled in cusps of the valve.

In one aspect, the heart valve is an aortic valve, and the method of the invention is a method of determining a plane of the aortic valve annulus. In one embodiment, the image is an X-ray image.

In another aspect, the invention is a method of implantation of a replacement heart valve into a diseased heart valve, the method comprising the steps of:
determining a plane of the diseased heart valve annulus according to a method of the invention,
withdrawing the locating device of the invention from the diseased heart valve;
positioning an undeployed replacement heart valve inside the diseased heart valve at an angle corresponding to the determined plane of the diseased heart valve annulus;
optionally, confirming the plane of the diseased heart valve annulus according to a method of the invention when the undeployed heart valve replacement has been positioned inside the diseased heart valve; and
deploying the replacement heart valve.

Other aspects and preferred embodiments of the invention are defined and described in the other claims set out below.

### Brief Description of the Figures

**Figure 1** is an illustration of the aorta and aortic valve showing the three aortic cusps.
**Figure 2** is a sectional view of the aorta of Figure 1 showing a replacement aortic valve implanted correctly with the frame of the replacement valve parallel with the annular plane of the aortic valve.
**Figure 3** is an illustration if the heart showing the aorta, aortic valve, left ventricle and right ventricle, with a cut-away showing the three aortic valve cusps disposed at the end of the aorta.
**Figure 4A and 4B** are illustrations of a health aortic valve looking towards the valve from the aorta, showing the valve in an open position (Fig. 4A) with the cusps stretched open, and in a closed position (Fig. 4B) with the cusps fully closed
**Figure 4C and 4D** are illustrations of a stenotic aortic valve looking towards the valve from the aorta, showing the valve in a partially open position (Fig. 4C) with the cusps restricted from opening fully due to calcification of the valve leaflets, and in a partially closed position (Fig. 4D) with the cusps restricted from closing fully due to calcification of the leaflets.
**Figure 5A** is a perspective view of a device of the invention showing the locating head in a deployed configuration distal of the delivery catheter.
**Figure 5B** is an end elevation view of the device of Fig. 5A looking towards a distal end of the device.
**Figure 5C** is an end elevation view of the device of Fig. 5A looking towards a proximal end of the device.
**Figure 6** is an illustration of a device of the invention in a deployed configuration with the loops of the locating head disposed within the aortic valve cusps.
**Figure 7** illustrates a use of the device of the invention in locating the plane of an aortic valve annulus during an aortic valve replacement procedure, in which:
   **Fig.7A** shows a guidewire advanced trans-femorally to the aorta, around the aortic arch, and through the aortic valve into the left ventricle;
   **Fig. 7B** shows a device of the invention in an undeployed configuration advanced over the guidewire to a position just proximal of the aortic valve;
   **Fig. 7C** shows the device in a deployed configuration with the three loops of the locating nestling in the cusps of the aortic valve with the apex of the loops abutting the base of the cusps. Once in this position, an X-ray picture is taken showing the location of the apex of the three loops from which an accurate 3-D image of the plane of the aortic valve annulus can be determined.
   **Fig. 7D** shows the aortic valve with the device of the invention retracted and withdrawn, and a replacement valve delivery catheter advanced over the guidewire through the aortic valve with the replacement valve in a contracted configuration spanning the valve;
   **Fig. 7E** is the same illustration as Fig. 7D but showing the replacement aortic valve in a radially-expanded deployed configuration; and
   **Fig. 7F** shows the valve replacement delivery catheter and guidewire withdrawn leaving the replacement valve in-situ in the aortic valve.
**Figure 8A** is a perspective view of an embodiment of the device of the invention in which the locating head has two separate cusp-locating loops, and **Figure 8B** is an end view of the device of Fig. 8A looking towards a distal end of the device.
**Figure 9A** is a perspective view of an embodiment of the device of the invention in which the three cusp-locating loops are attached to the control shaft by extension arms, and
**Figure 9B** is an end view of the device of Fig. 9A looking towards a distal end of the device.
**Figure 10A** is a perspective view of an embodiment of the device of the invention in which the three cusp-locating loops are attached to the control shaft by extension arms and in which the cusp-locating loops are hook-shaped, and **Figure 10B** is an end view of the device of Fig. 10A looking towards a distal end of the device.
**Figure 11A** is a perspective view of an embodiment of the device of the invention in which the cusp-locating heads are atraumatic spheres, and **Figure 11B** is an end view of the device of Fig. 11A looking towards a distal end of the device.
**Figure 12A** is a perspective view of an embodiment of the device of the invention in which the locating head has two cusp-locating heads provided by atraumatic spheres, and **Figure 12B** is an end view of the device of Fig. 12A looking towards a distal end of the device.

### Detailed Description of the Invention

All publications, patents, patent applications and other references mentioned herein are hereby incorporated by reference in their entireties for all purposes as if each individual publication, patent or patent application were specifically and individually indicated to be incorporated by reference and the content thereof recited in full.

### Definitions and general preferences

Where used herein and unless specifically indicated otherwise, the following terms are intended to have the following meanings in addition to any broader (or narrower) meanings the terms might enjoy in the art:
Unless otherwise required by context, the use herein of the singular is to be read to include the plural and *vice versa.* The term "a" or "an" used in relation to an entity is to be read to refer to one or more of that entity. As such, the terms "a" (or "an"), "one or more," and "at least one" are used interchangeably herein.

As used herein, the term "comprise," or variations thereof such as "comprises" or "comprising," are to be read to indicate the inclusion of any recited integer (e.g. a feature, element, characteristic, property, method/process step or limitation) or group of integers (e.g. features, element, characteristics, properties, method/process steps or limitations) but not the exclusion of any other integer or group of integers. Thus, as used herein the term "comprising" is inclusive or open-ended and does not exclude additional, unrecited integers or method/process steps.

As used herein, the term "disease" is used to define any abnormal condition that impairs physiological function and is associated with specific symptoms. The term is used broadly to encompass any disorder, illness, abnormality, pathology, sickness, condition or syndrome in which physiological function is impaired irrespective of the nature of the aetiology (or indeed whether the aetiological basis for the disease is established). It therefore encompasses conditions arising from infection, trauma, injury, surgery, radiological ablation, poisoning or nutritional deficiencies.

As used herein, the term "treatment" or "treating" refers to an intervention (e.g. the administration of an agent to a subject) which cures, ameliorates or lessens the symptoms of a disease or removes (or lessens the impact of) its cause(s) (for example, the reduction in accumulation of pathological levels of lysosomal enzymes). In this case, the term is used synonymously with the term "therapy".

Additionally, the terms "treatment" or "treating" refers to an intervention (e.g. the administration of an agent to a subject) which prevents or delays the onset or progression of a disease or reduces (or eradicates) its incidence within a treated population. In this case, the term treatment is used synonymously with the term "prophylaxis".

In the context of treatment and effective amounts as defined above, the term *subject* (which is to be read to include "individual", "animal", "patient" or "mammal" where context permits) defines any subject, particularly a mammalian subject, for whom treatment is indicated. Mammalian subjects include, but are not limited to, humans, domestic animals, farm animals, zoo animals, sport animals, pet animals such as dogs, cats, guinea pigs, rabbits, rats, mice, horses, cattle, cows; primates such as apes, monkeys, orangutans, and chimpanzees; canids such as dogs and wolves; felids such as cats, lions, and tigers; equids such as horses, donkeys, and zebras; food animals such as cows, pigs, and sheep; ungulates such as deer and giraffes; and rodents such as mice, rats, hamsters and guinea pigs. In preferred embodiments, the subject is a human.

"Plane of the heart valve annulus" refers to a plane formed between the base of the three valve cusps, indicated in Figure 1 with the letter E. One purpose of the invention is to determine the plane using a device of the invention and provide an X-ray image of the plane that can be viewed in real time on a catherization lab table. This image can then be used to correctly position a replacement heart valve within the native diseased aortic valve prior to deployment, with a plane defined by the base of the replacement heart valve aligned with the plane of the heart valve annulus. It will be appreciated that the position of the heart valve annulus will probably change after the image is taken, due to the beating of the heart or movement of the lungs, however while the position of the annulus may change, the plane of the annulus tends to remain the same.

"Cusp-locating heads" refers to the parts of the locating head which are configured upon deployment to nestle within the cusps of a heart valve, especially the aortic valve cusps. In a preferred embodiment, the cusp-locating heads are adjacent, unconnected, loops having a distal end that is typically dimensioned to nestle in a cusp of the heart valve. The term "unconnected" as applied to the loops means that adjacent loops and not intertwined, and can move independently of each other. This is important as it allows for positional adjustment of the loops relative to each other to allow the loops engage with cusps having different dimensions and different anatomy. In other embodiments, the cusp-locating heads may be loops provided on the ends of extension arms, complete and incomplete loops, discs, spherical heads, or any element that is shaped to nestle within a cusp. The loops are generally inwardly facing, as shown in Figs. 95B, 9B and 10B. The cusp-locating heads angle generally outwardly relative to an axis of the control shaft, for example at an angle of 20-60°, for example 30-40°, and it will be appreciated that the angle may vary depending on the length of the cusp-locating heads. The heads may taper slightly inwardly, in a proximal to distal direction, or they may be substantially straight.

"Radially adjustable" as applied to the cusp-locating head means that upon deployment the cusp-locating heads angle outwardly to assume a trifurcated cusp-locating position (i.e. tripod geometry). In the case of devices for bicuspid valves, the cusp-locating heads angle outwardly to assume a bifurcated position. Generally, the locating head will be formed from a shape-memory material (for example, nitinol) which biases the head into the trifurcated or bifurcated orientation, upon release of a restraint (for example, advancing of the head beyond the end of the end of the delivery catheter, or withdrawal of the catheter). Shape memory polymers are described in Lendlein et al (Angew. Chem. Int. Ed. 41(12):2034-2057) and Chan et al (Recent Advances in Shape Memory Soft Materials for Biomedical Applications". ACS Applied Materials & Interfaces. 8 (16): 10070-10087). However, embodiments of the device are envisaged where the head is not formed of a shape-memory material, and deployment of the device involves manual deployment of the locating head using control arms of the like.

"Radiopaque material" or "radiopaque marker" in the context of the present application refers to part of the apex of the cusp-engaging head which comprises a dense material which is translucent to X-radiation, and therefore can be imaged under X-ray. Radiopaque marker bands for medical devices are provided by Putnam Plastics (CT, USA) and generally comprise a highly loaded tungsten-filled thermoplastic polymer that can be provided in many shaped and sizes. Radiopaque marker coating are provided by ProPlate (MN, USA).

"Shaped to nest snugly within an aortic valve cusp" as applied to the cusp-locating head should be understood to mean that the head is dimensioned to conform to the geometry of the valve cusp. Generally, this means that the cusp-engaging head is curved, for example the apex of a loop, and ensures that the head fits within the cusp and closely conforms to the base of the cusp.

### Exemplification

The invention will now be described with reference to specific Examples. These are merely exemplary and for illustrative purposes only: they are not intended to be limiting in any way to the scope of the monopoly claimed or to the invention described. These examples constitute the best mode currently contemplated for practicing the invention.

Figure 1 is an illustration of the aorta A, left ventricular outflow tract B, and aortic valve C and its three aortic cusps D1 to D3. The annular plane of the valve is indicated by the broken line E. Figure 2 is a sectional view of the aorta of Figure 1 showing a replacement aortic valve 1 implanted correctly with the frame of the replacement valve 2 located in a plane parallel to the annular plane E of the aortic valve.

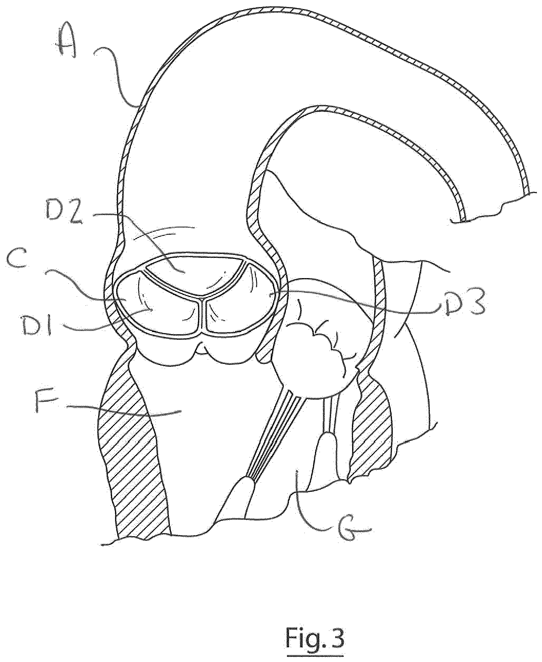

Figure 3 is an illustration of the heart showing the aorta A, aortic valve C, left ventricle F and right ventricle G, with a cut-away showing the three aortic valve cusps D1 to D3 of the aortic valve C. It can be seen from Figure 3 that the aortic valve cusps when closed re concave in shape.

Figure 4A and 4B are illustrations of a health aortic valve C looking towards the valve from the aorta A, showing the valve in an open position (Fig. 4A) with the cusps D1 to D3 flattened, and in a closed position (Fig. 4B) with the cusps fully opened. When in a close configuration, the cusps are flattened into pockets.

Figure 4C and 4D are illustrations of a stenotic aortic valve looking towards the valve from the aorta, showing the valve in a partially open position (Fig. 4C) with the cusps restricted from opening fully due to calcification of the valve leaflets, and in a partially closed position (Fig. 4D) with the cusps restricted from closing fully due to calcification of the leaflets.

Figures 5A - 5B show a device of the invention in a deployed configuration, indicated generally by the reference numeral 5, and comprising a delivery catheter 6 having an internal lumen 7, a control shaft 8, and a locating head disposed at a distal end of the control shaft, comprising three cusp locating heads, in this embodiment provided by loops 9. The three loops 9 are connected to the control shaft at their proximal ends, and angle outwardly from an axis of the central shaft at an angle of about 30-40 degrees. The loops 9 are not interconnected or intertwined, but are separate from each other and configured for movement independently of each other. The apex 10 of each loop is dimensioned to fit into a cusp of an aortic valve, and comprises a radio-opaque marker 11 (for example a coating of a noble metal such as gold). The control shaft 8 and locating head are axially movable relative to the catheter from a stowed position within the lumen of the catheter, to a deployed position shown in Fig 5 in which the locating head is deployed distally of the distal end of the catheter. Although not shown, the proximal end of the catheter may include a handle comprising actuation means for deploying and retracting the control shaft and locating head.

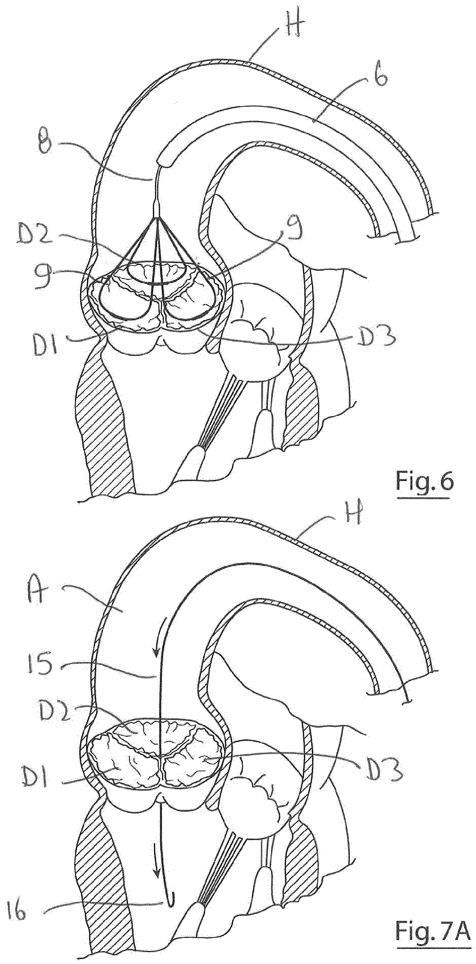

Figure 6 is an illustration of the device of Fig. 5 in use, and shows the device 5 in a deployed configuration with the loops 9 of the locating head disposed within the aortic valve cusps D1 to D3. When in this position, the apex of each loop will be positioned at the base of a cusp, and an X-Ray image can be taken which will show, in 3-D, the position of the apex of each loop due to the radio-opaque marker disposed on the apex of each cusp.

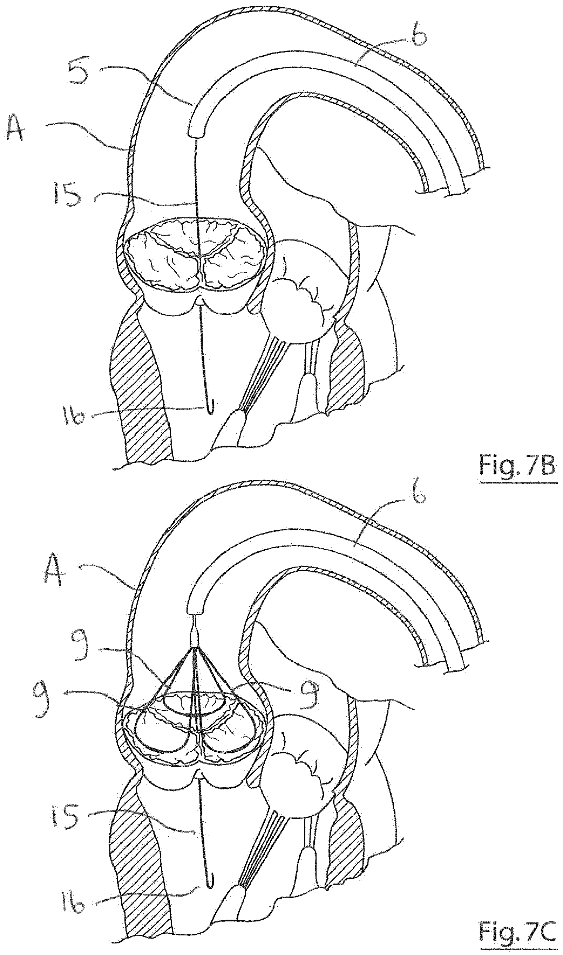

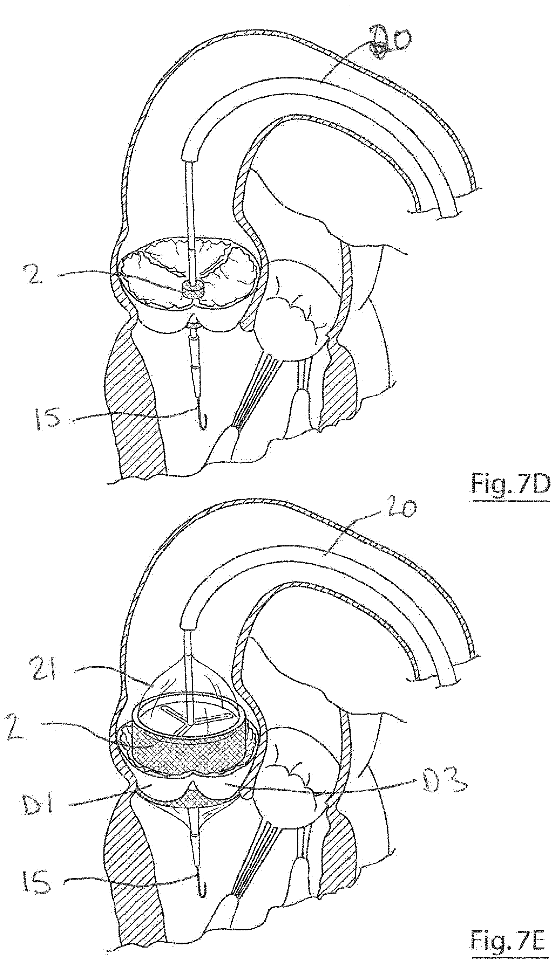

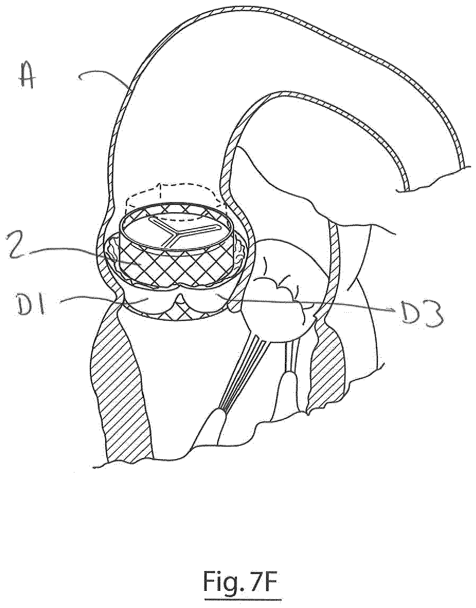

Figure 7 illustrates a method of the invention that employs a locating device of the invention, specifically a method of locating the plane of an aortic valve annulus during an aortic valve replacement procedure. In will be appreciated that the method exemplified is one possible method of using the device of the invention, and that the device of the invention may be employed with other valve replacement procedures, including for examples procedures in which the aortic valve is opened by a balloon prior to insertion of the replacement valve.

In a first step, and referring to Fig.7A, a guidewire 15 with an atraumatic head 16 is advanced transfemorally to the aorta A, around the aortic arch H, and through the aortic valve C (shown in a closed position) into the left ventricle F via left ventricular outflow tract B. Fig. 7B shows a device of the invention 5 in an undeployed configuration advanced over the guidewire 15 to a position just proximal of the aortic valve C. The device is then deployed with the locating head being advanced distally of the end of the catheter - Fig. 7C shows the device with the three loops 9 of the locating head advanced and positionally adjusted until the loops nestle in the cusps D1 to D3 of the aortic valve with the apex 10 of the loops abutting the base of the cusps. Once in this position, an X-ray picture is taken showing the location of the radiopaque marker 11 at the apex 10 of the three loops, which represents an accurate 3-D image of the plane of the aortic valve annulus, and this image is saved and displayed on the screen in the cath lab. Once the image has been taken, the shaft and locating head is retracted into the catheter, and the catheter is withdrawn along the guidewire. In some embodiments, the catheter may not be fully withdrawn.

As shown in Fig. 7D, a replacement valve catheter 20 carrying a replacement valve 2 mounted on an inflatable balloon 21 is advanced along the aorta over the guidewire, and advanced through the aortic valve and height adjusted so that the distal end of the valve is positioned just distal of the aortic valve annulus. The operator then delivers the TAVI valve in the plane determined by the device of the invention. The heads/loops of the device of the invention catheter are then generally re-advanced into the aortic sinuses to provide a real-time assessment of the position of the annular plane and the final position of the valve with respect to the annular plane is determined. The heads/loops of the device of the invention are then withdrawn and the TAVI valve is deployed Fig. 7F shows the replacement valve delivery catheter and guidewire withdrawn leaving the replacement valve in-situ in the aortic valve.

Figures 8A and 8B illustrate an alternative embodiment of the device of the invention, in which parts described previously are assigned the same reference numerals. In this embodiment, the locating head has two cusp-locating loops 29 as opposed to three, and is designed for use with patients having bicuspid aortic valves. An apex of the loops 29 is coated with a radio-opaque coating 11 to allow visualisation under X-ray imaging. The use of this device is the same as the device having a trifurcated locating head described above.

Figures 9A and 9B illustrate an alternative embodiment of the device of the invention, in which parts described previously are assigned the same reference numerals. In this embodiment, the locating head of the device comprises three extension arms 30 each having a cusp-locating loop 31 at a distal end thereof. The extension arms 30 angle outwardly of a longitudinal axis of the device, and the loops are configured to nest within the cusps of the aortic valve. An apex of the loops 31 is coated with a radio-opaque coating 11 to allow visualisation under X-ray imaging. The use of this device is the same as the device having a trifurcated locating head described above.

Figures 10A and 10B illustrate an alternative embodiment of the device of the invention, in which parts described previously are assigned the same reference numerals. In this embodiment, the locating head of the device comprises three extension arms 30 each having a cusp-locating loop 32 at a distal end thereof. The extension arms 30 angle outwardly of a longitudinal axis of the device, similar to the device of Fig. 9, and the loops 32 are configured to nest within the cusps of the aortic valve. In this embodiment, the loops are generally hook shaped. An apex of the loops 32 is coated with a radio-opaque coating to allow visualisation under X-ray imaging. The use of this device is the same as the device having a trifurcated locating head described above.

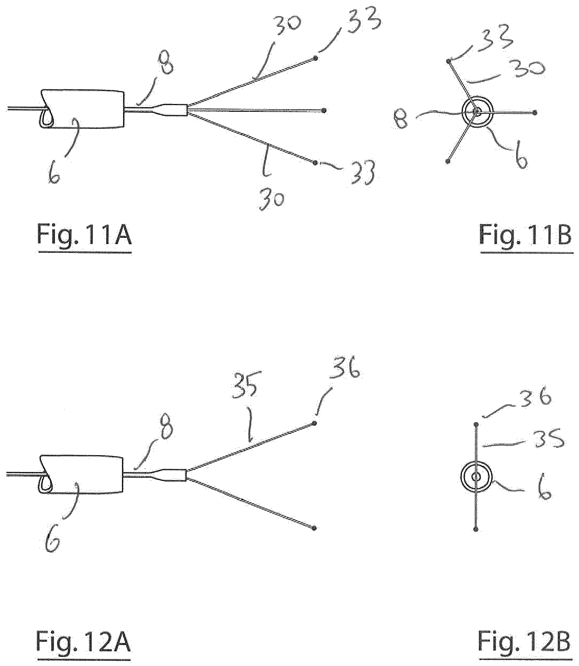

Figures 11A and 11B illustrate an alternative embodiment of the device of the invention, in which parts described previously are assigned the same reference numerals. In this embodiment, the locating head of the device comprises three extension arms 30 each having a cusp-locating sphere 33 at a distal end thereof. The extension arms 30 angle outwardly of a longitudinal axis of the device, and the spheres 33 are configured to nest within the cusps of the aortic valve and are coated with a radio-opaque coating to allow visualisation under X-ray imaging. The use of this device is the same as the device having a trifurcated locating head described above.

Figures 12A and 12B illustrate an alternative embodiment of the device of the invention, in which parts described previously are assigned the same reference numerals. In this embodiment, the locating head has two cusp-locating heads, in this case provided by extension arms 35 and distal spheres 36 coated with radio-opaque coating. This embodiment is designed for use with patients having bicuspid aortic valves. The use of this device is the same as the device having a trifurcated locating head described above.

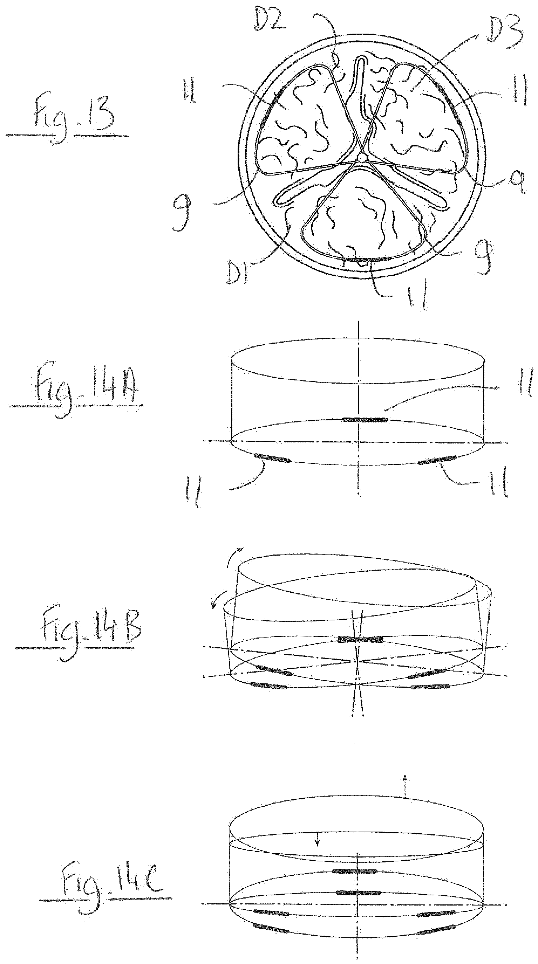

Figure 13 illustrates a device of the invention in-situ within the cusps of an aortic valve, shown from above the valve. Figures 14A - 14C are illustrations of an X-ray image of the aortic valve with a device of the invention in-situ as shown in Fig. 13, and how the three radio-opaque markers disposed on the apex of the loops are visualised enabling a 3-D representation of the plane of the aortic valve annulus be determined on X-ray. Figure 14B shows the valve sitting

### Equivalents

The foregoing description details presently preferred embodiments of the present invention. Numerous modifications and variations in practice thereof are expected to occur to those skilled in the art upon consideration of these descriptions. Those modifications and variations are intended to be encompassed within the claims appended hereto.

## Claims

1. A device (5) for locating a plane of an aortic valve annulus (E), comprising an elongated control shaft (8) and a locating head at a distal end of the shaft, the locating head comprising three adjacent and separate cusp-locating loops (9) that are radially adjustable from a delivery configuration to a deployed trifurcated configuration where the cusp-locating loops (9, 31, 32) are angled outwardly relative to the central shaft and positioned to nest in the cusps (D1, D2, D3) of a stenotic aortic valve during use, wherein an apex (10) of each cusp-locating head comprises a radio-opaque material (11).

2. A device as claimed in Claim 1, in which the cusp-locating loop is a closed loop (9, 31).

3. A device as claimed in Claim 1 or 2, in which the cusp-locating loop comprises an extension arm (30).

4. A device as claimed in any preceding Claim in which an apex (10) of each loop when deployed is shaped to self-centre the loop within an aortic valve cusp (D1, D2, D3).

5. A device as claimed in any preceding Claim, in which the loops (9, 31, 32) are formed from a shape-memory material.

6. A device as claimed in any preceding Claim, and including an elongated catheter (6) having a central lumen (7), wherein the control shaft (8) and locating head are disposed within the lumen of the catheter and axially movable relative to the catheter to deploy the locating head.

7. A device as claimed in any preceding Claim, in which each loop has a length of X-Y mm, and an apex of each loop has a width of X-Y mm.

8. A device as claimed in any preceding Claim, in which the cusp-locating loops are angled outwardly relative to the central shaft at an angle of 25-60°.
